# EUROPEAN PATENT APPLICATION

(11) **EP 4 733 828 A1**
(43) Date of publication of application: **29.04.2026**
(21) Application number: 24830440.4
(22) Date of filing: 06.06.2024
(51) Int. Cl.: G02B 27/09, H01S 5/06, A61B 18/20

(54) **VARIABLE LIGHT SPOT LENS STRUCTURE AND LASER DEVICE**

(30) Priority: 25.06.2023 CN 202321619753 U
(71) Applicant: Focuslight Technologies Inc., Xi'an, Shaanxi 710077 (CN)
(72) Inventor: QU, Jiaojiao, Xi'an, Shaanxi 710077 (CN); CAI, Lei, Xi'an, Shaanxi 710077 (CN); LI, Zhi, Xi'an, Shaanxi 710077 (CN); CAO, Heng, Xi'an, Shaanxi 710077 (CN)
(74) Representative: Fritz Patent- und Rechtsanwälte PartmbB
(86) International application number: PCT/CN2024/097675
(87) International publication number: WO 2025/001794

(57) **Abstract**

A variable spot lens structure and a laser device. The variable spot lens structure comprises a fixed module (10) and a plurality of replacement modules (20). The fixed module (10) comprises a fixed base (11), a light source (12) disposed inside the fixed base (11), a fast-axis collimator disposed on a light-emitting side of the light source (12), and a compression unit (13) fixed on the fixed base (11) and located on a light-emitting side of the fast-axis collimator. The plurality of replacement modules (20) are interchangeably disposed on a light-emitting side of the fixed module (10), and each replacement module (20) comprises an optical head (21) and a shaping unit. The optical head (21) of each replacement module (20) is detachably connectable to the fixed base (11) of the fixed module (10). A light beam emitted from the light source (12) is incident on the replacement module (20) after fast-axis compression by the fast-axis collimator and compression by the compression unit (13), and is emitted after being shaped by the shaping unit of the replacement module (20). Spot sizes of light beams emitted through any two different replacement modules (20) on a receiving surface are different. Such a variable spot lens structure can output multiple specifications of small-sized spots, improving the application flexibility of laser modules.

## Description

### Technical field

The invention relates to the field of optical technology, and in particular, to a variable spot lens structure and a laser device.

### Background

High-power semiconductor lasers have advantages such as small size, light weight, high efficiency, and long service life, and have been widely used in industrial processing, cladding, pumping, medical fields, etc., becoming one of the core devices with the fastest development, most achievements, extensive discipline penetration, and broad application scope in the new century.

In the field of medical aesthetics, the main applications of lasers are spot removal, hair removal, etc. However, on one hand, the lens structures used in existing laser devices can only output spots of fixed specifications, and the spot size cannot be adjusted, which greatly restricts the application of semiconductor laser devices. On the other hand, it is difficult for existing laser devices to emit small-sized spots, which particularly limits their application scenarios for laser devices used in fields such as spot removal and hair removal.

### Summary of the invention

The purpose of the invention is to provide a variable spot lens structure and a laser device. The variable spot lens structure and the laser device can output multiple different specifications of small-sized spots, improving the application flexibility of laser modules.

Embodiments of the invention are implemented as follows:
In one aspect, the invention provides a variable spot lens structure. The variable spot lens structure comprises a fixed module and a plurality of replacement modules configured to cooperate with the fixed module. The fixed module comprises a fixed base, a light source disposed inside the fixed base, a fast-axis collimator disposed on a light-emitting side of the light source, and a compression unit fixed on the fixed base and located on a light-emitting side of the fast-axis collimator. The plurality of replacement modules are used for being interchangeably disposed on a light-emitting side of the fixed module, and each of the replacement modules comprises an optical head and a shaping unit disposed inside the optical head. The optical head of each replacement module is detachably connectable to the fixed base of the fixed module. A light beam emitted from the light source is incident on the replacement module after fast-axis compression by the fast-axis collimator and compression by the compression unit and is emitted after being shaped by the shaping unit of the replacement module. Spot sizes of light beams emitted through any two different replacement modules on a receiving surface are different.

This variable spot lens structure can output multiple specifications of small-sized spots, improving the application flexibility of laser modules.

Optionally, the compression unit comprises lenses or lens groups arranged sequentially along an optical axis direction.

Optionally, the fixed module further comprises a first pressing frame. The first pressing frame is disposed on a side of the fixed base close to the replacement module and is used for fixing the compression unit to the fixed base.

Optionally, the fixed base comprises a mounting seat and a mounting head connected to the mounting seat. The light source is disposed inside the mounting seat. The compression unit is fixed on the mounting seat. The optical head of the replacement module is detachably connected to the mounting head.

Optionally, the fixed module further comprises a thermoelectric cooler. The mounting head is connected to the mounting seat through the thermoelectric cooler. A hot side of the thermoelectric cooler is close to the mounting seat, and a cold side thereof is close to the mounting head. Optionally, a cooling channel is provided inside the mounting seat. Optionally, the shaping unit comprises at least one optical lens. The replacement module further comprises a second pressing frame. The second pressing frame is disposed on a side of the replacement module close to the fixed module and is used for fixing the optical lens inside the optical head.

Optionally, the shaping unit comprises a light-guiding element. Optionally, the light-guiding element is a light guide rod. The light guide rod penetrates through the optical head and is located on a light-emitting side of the optical lens. A light beam emitted from the optical lens can be emitted after passing through the light guide rod.

Optionally, the light-guiding element is a reflective cavity. The reflective cavity penetrates through the optical head. A light beam emitted from the fixed module can be emitted after being reflected by the reflective cavity. Optionally, the fixed base and the optical head are connected by a hinge; or, the fixed base and the optical head are screw-connected; or, the fixed base is provided with a guide groove, the optical head is provided with a guide rail adapted to the guide groove, and the guide rail is slidably insertable into the guide groove; or, the fixed base is provided with a first engagement portion, the optical head is provided with a second engagement portion, and the first engagement portion and the second engagement portion are engaged with each other; or, a surface of the fixed base close to the optical head is provided with a first magnet, a surface of the optical head close to the fixed base is provided with a second magnet, and the first magnet and the second magnet are capable of attracting each other; or, the fixed base is provided with a first thread, the optical head is provided with a second thread, and the first thread and the second thread are threadedly connected.

Optionally, a surface of the optical head close to the fixed base is provided with two positioning pins, a surface of the fixed base close to the optical head is provided with two positioning holes, and the two positioning pins and the two positioning holes correspond one-to-one; wherein, diameters of the two positioning pins are different, or, the two positioning pins are arranged asymmetrically about a center of the optical head.

Optionally, the variable spot lens structure further comprises an identification module and a controller. The identification module is used for identifying a type of the replacement module and transmitting it to the controller. The controller is used for controlling an output light energy of the light source according to the type of the replacement module.

Optionally, the identification module comprises a plurality of probes disposed on a surface of the fixed base close to the optical head. Each optical head of the replacement modules is provided with a plurality of probe holes corresponding one-to-one with the probes. When an optical head of a different replacement module is connected to the fixed base of the fixed module, two different probes can be electrically connected and send an identification signal to the controller. The identification signal is different when different replacement modules are connected to the fixed module. The controller can determine the type of the replacement module connected to the fixed module according to the identification signal.

Optionally, the identification module comprises a pressure sensor disposed on a surface of the fixed base close to the optical head. Each optical head of the replacement modules is provided with a different engagement portion. When the optical head of the replacement module is connected to the fixed base of the fixed module, the engagement portion presses the pressure sensor. The pressure sensor can send a corresponding pressure signal to the controller. The pressure signal is different when different replacement modules are connected to the fixed module. The controller can determine the type of the replacement module connected to the fixed module according to the pressure signal. Optionally, the identification module comprises a signal generator and a detector disposed on the fixed base. Each optical head of the replacement modules is provided with a reflector, and a distance from the reflector of each replacement module to the fixed base is different. When the optical head of the replacement module is connected to the fixed base of the fixed module, the signal generator emits a signal to the reflector and the signal is reflected by the reflector and then received by the detector. The controller is used for obtaining a detection signal from the detector and determining the type of the replacement module connected to the fixed module according to the detection signal.

In another aspect, the invention provides a laser device comprising the aforementioned variable spot lens structure.

### Beneficial Effects

The beneficial effects of the invention include:
The variable spot lens structure provided in this application comprises a fixed module and a plurality of replacement modules configured to cooperate with the fixed module. The fixed module comprises a fixed base, a light source disposed inside the fixed base, a fast-axis collimator disposed on a light-emitting side of the light source, and a compression unit fixed on the fixed base and located on a light-emitting side of the fast-axis collimator. The plurality of replacement modules are used for being interchangeably disposed on a light-emitting side of the fixed module, and each of the replacement modules comprises an optical head and a shaping unit disposed inside the optical head. The optical head of each replacement module is detachably connectable to the fixed base of the fixed module. A light beam emitted from the light source is incident on the replacement module after fast-axis compression by the fast-axis collimator and compression by the compression unit and is emitted after being shaped by the shaping unit of the replacement module. Spot sizes of light beams emitted through any two different replacement modules on a receiving surface are different. In this application, by equipping the fixed module with multiple different replacement modules, one set of the variable spot lens structure can achieve output of spots with multiple specification sizes, meeting various needs of users, and having good market application prospects. Compared to existing laser devices that can only output one size of spot, the flexibility of this application is better.

Furthermore, the fixed module in this application is provided with the fast-axis collimator and the compression unit, so that within the fixed module, this application can reduce the divergence angle of the light beam emitted by the light source in the fast-axis direction, making the fast-axis and slow-axis divergence angles similar. While making the fast-axis and slow-axis divergence angles similar, this application also compresses the light beam through the compression unit, thereby enabling small-sized spots to be obtained on the receiving surface.

Compared to arranging the compression unit inside the replacement module, this can further reduce the number of optical elements in the variable spot lens structure, reduce the overall volume of the variable spot lens structure, and achieve cost reduction. Thus, the variable spot lens structure provided in this application can output multiple different specifications of small-sized spots, improving the application flexibility of laser modules.

### Brief Description of Drawings

To illustrate the technical solutions of the embodiments of the invention more clearly, the following will briefly introduce the drawings required for describing the embodiments. It should be understood that the following drawings only show some embodiments of the invention, and therefore should not be considered as limiting the scope. For those of ordinary skill in the art, other related drawings can be obtained based on these drawings without creative effort.
FIG. 1 is a first schematic structural diagram of a variable spot lens structure provided by an embodiment of the invention.
FIG. 2 is a second schematic structural diagram of the variable spot lens structure provided by an embodiment of the invention.
FIG. 3 is a third schematic structural diagram of the variable spot lens structure provided by an embodiment of the invention.
FIG. 4 is a schematic structural diagram of a fixed module provided by an embodiment of the invention.
FIG. 5 is a first schematic structural diagram of a replacement module provided by an embodiment of the invention.
FIG. 6 is an axonometric view of FIG. 5.
FIG. 7 is a second schematic structural diagram of a replacement module provided by an embodiment of the invention.
FIG. 8 is a schematic structural diagram of a replacement module with a reflective cavity.
FIG. 9 is an axonometric view of FIG. 7.
FIG. 10 is a third schematic structural diagram of a replacement module provided by an embodiment of the invention.
FIG. 11 is an axonometric view of FIG. 10.
FIG. 12 is a first schematic structural diagram of an optical head provided by an embodiment of the invention.
FIG. 13 is a second schematic structural diagram of an optical head provided by an embodiment of the invention.
FIG. 14 is a schematic structural diagram of a mounting head provided by an embodiment of the invention.
FIG. 15 is a schematic structural diagram of an optical head provided by an embodiment of the invention.

### Reference numerals:

10 - fixed module
11 - fixed base
111 - mounting seat
112 - mounting head
113 - positioning hole
12 - light source
13 - compression unit
131 - positive lens
14 - first spacer ring
15 - first pressing frame
16 - thermoelectric cooler
20 - replacement module
21 - optical head
211 - positioning pin
212 - probe hole
221 - optical lens
222 - light guide rod
223 - reflective cavity
23 - second pressing frame
24 - second spacer ring
25 - window plate
31 - probe.

### Detailed Description

To make the purposes, technical solutions, and advantages of the embodiments of the invention clearer, the technical solutions in the embodiments of the invention will be described clearly and completely with reference to the accompanying drawings in the embodiments of the invention. Obviously, the embodiments described are only part of the embodiments of the invention, not all of them. The components of the embodiments of the invention generally described and illustrated in the drawings herein can be arranged and designed in various configurations. Therefore, the following detailed description of the embodiments of the invention provided in the accompanying drawings is not intended to limit the scope of the claimed invention but merely represents selected embodiments of the invention. Based on the embodiments of the invention, all other embodiments obtained by those of ordinary skill in the art without creative effort shall fall within the protection scope of the invention.

It should be noted that similar reference numerals and letters indicate similar items in the following drawings. Therefore, once an item is defined in one drawing, it does not need to be further defined and explained in subsequent drawings.

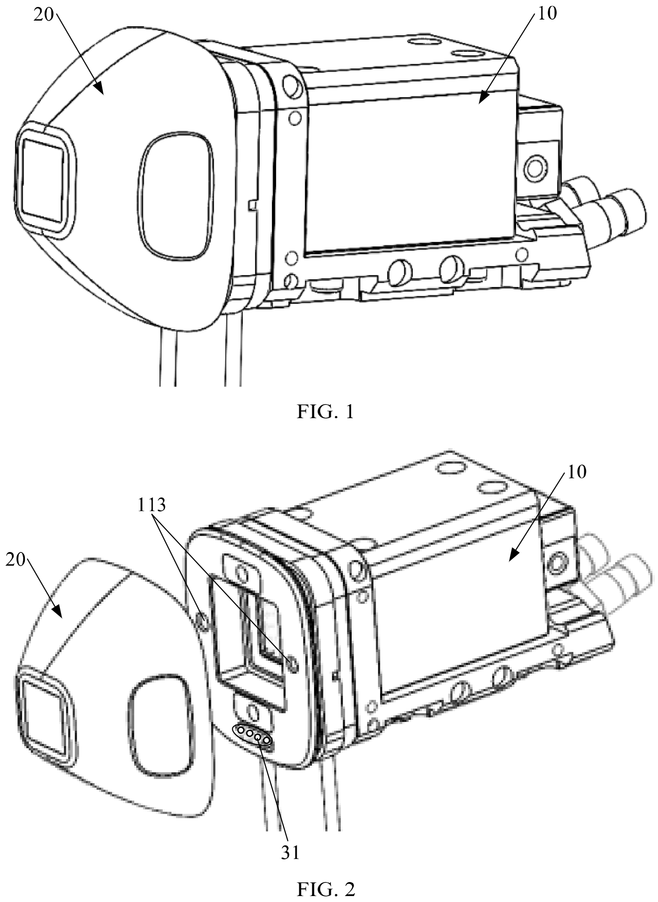

In the description of the invention, it should be noted that the orientation or positional relationships indicated by terms such as "center," "upper," "lower," "left," "right," "vertical," "horizontal," "inner," "outer," etc., are based on the orientation or positional relationships shown in the drawings, or the orientation or positional relationships in which the product of the invention is conventionally placed when in use. They are only for the convenience of describing the invention and simplifying the description, and do not indicate or imply that the referred device or element must have a specific orientation or be constructed and operated in a specific orientation. Therefore, they should not be construed as limiting the invention. In addition, terms such as "first," "second," "third," etc., are used only for distinguishing description and should not be construed as indicating or implying relative importance. Furthermore, terms such as "horizontal," "vertical," etc., do not require that the components be absolutely horizontal or vertical, but may be slightly inclined. For example, "horizontal" merely means that its direction is more horizontal relative to "vertical," and does not mean that the structure must be completely horizontal; it may be slightly inclined. In the description of the invention, it should also be noted that unless otherwise explicitly specified and limited, the terms "disposed," "installed," "connected," and "linked" should be interpreted broadly. For example, they may be fixed connections, detachable connections, or integral connections; they may be mechanical connections, electrical connections, or direct connections, or indirect connections through an intermediate medium, or internal communication between two elements. For those of ordinary skill in the art, the specific meanings of the above terms in the invention can be understood based on the specific context. Referring to FIG. 1 to FIG. 4, this embodiment provides a variable spot lens structure. The variable spot lens structure comprises a fixed module 10 and a plurality of replacement modules 20 configured to cooperate with the fixed module 10. The fixed module 10 comprises a fixed base 11, a light source 12 disposed inside the fixed base 11, a fast-axis collimator disposed on a light-emitting side of the light source 12, and a compression unit 13 fixed on the fixed base 11 and located on a light-emitting side of the fast-axis collimator. The plurality of replacement modules 20 are used for being interchangeably disposed on a light-emitting side of the fixed module 10, and each of the replacement modules 20 comprises an optical head 21 and a shaping unit disposed inside the optical head 21. The optical head 21 of each replacement module 20 is detachably connectable to the fixed base 11 of the fixed module 10. A light beam emitted from the light source 12 is incident on the replacement module 20 after fast-axis compression by the fast-axis collimator and compression by the compression unit 13 and is emitted after being shaped by the shaping unit of the replacement module 20. Spot sizes of light beams emitted through any two different replacement modules 20 on a receiving surface are different. This variable spot lens structure can output multiple specifications of small-sized spots, improving the application flexibility of laser modules.

It should be noted that: First, referring to FIG. 1 and FIG. 2, the variable spot lens structure provided in this application comprises a fixed module 10 and a plurality of replacement modules 20, wherein the plurality of replacement modules 20 can all cooperate with the fixed module 10, i.e., the plurality of replacement modules 20 are respectively used in cooperation with the fixed module 10. For example, a first replacement module 20 can cooperate with the fixed module 10, a second replacement module 20 can also cooperate with the fixed module 10, ... (and so on for other replacement modules 20).

In this embodiment, each replacement module 20 is detachably connectable to the fixed module 10. Thus, users can select a suitable replacement module 20 to install and cooperate with the fixed module 10 according to their needs, thereby achieving spot output. When a spot of another size needs to be output, the currently cooperating replacement module 20 can be detached from the fixed module 10, and then a replacement module 20 capable of outputting the corresponding spot size can be detachably installed on the fixed module 10.

Also, it should be noted that in this embodiment, the spot sizes of the light beams emitted through any two different replacement modules 20 on the receiving surface are different. Taking the plurality of replacement modules 20 including a first replacement module 20 and a second replacement module 20 as an example, when the first replacement module 20 is installed on the fixed module 10, a first spot can be obtained on the receiving surface; when the second replacement module 20 is installed on the fixed module 10, a second spot can be obtained on the receiving surface; the sizes of the first spot and the second spot are different.

In this application, by equipping the fixed module 10 with multiple different replacement modules 20, one set of the variable spot lens structure can achieve output of spots with multiple specification sizes, meeting various needs of users, and having good market application prospects. Compared to existing laser devices that can only output one size of spot, the flexibility of this application is better.

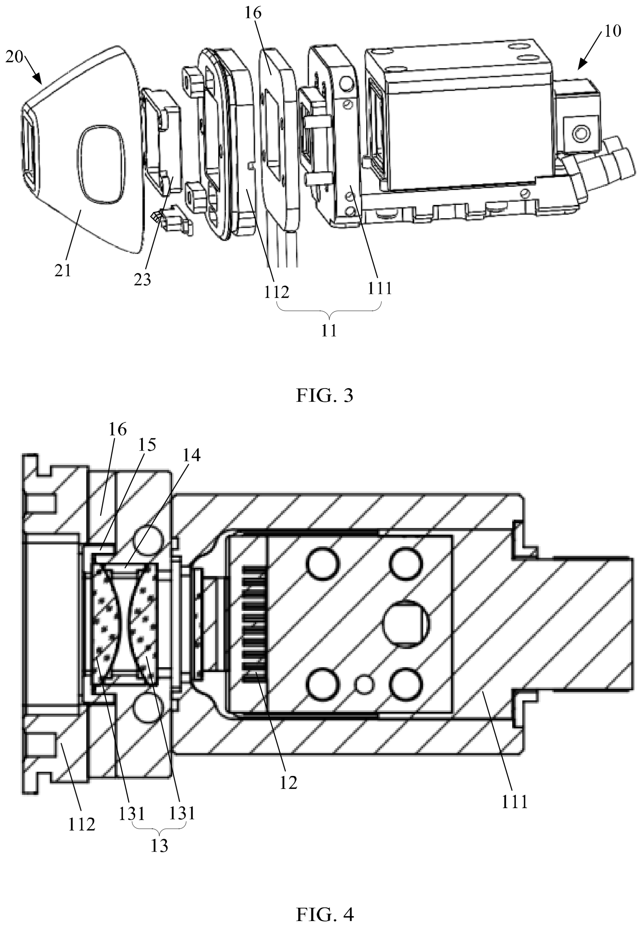

Second, referring to FIG. 3 and FIG. 4, the fixed module 10 comprises a fixed base 11, a light source 12, a fast-axis collimator, and a compression unit 13. The light source 12 is disposed inside the fixed base 11. The fast-axis collimator is disposed on the light-emitting side of the light source 12 and is used for compressing the light beam in the fast-axis direction to reduce the difference between the divergence angle of the light beam emitted from the light source 12 in the fast-axis direction and its divergence angle in the slow-axis direction. The compression unit 13 is fixed on the fixed base 11, and the light beam emitted from the light source 12 can be incident on the compression unit 13. The compression unit 13 is used for compressing the light beam emitted from the light source 12 and then emitting it. Thus, the light beam emitted from the fixed module 10 can be compressed to some extent. Therefore, the spot obtained on the receiving surface after passing through the replacement module 20 can be compressed, and thus the spot size obtained on the receiving surface can be reduced. In this way, small-sized spots can be output through the variable spot lens structure provided by this application.

This application does not limit the optical element form of the compression unit 13. For example, optionally, the compression unit 13 can comprise lenses or lens groups arranged sequentially along the optical axis direction. The lens can be a positive lens 131, or it can be two positive lenses 131. For example, when the compression unit 13 comprises two positive lenses 131, the fixed module 10 further comprises a first spacer ring 14. The first spacer ring 14 is embedded in a first accommodating cavity of the fixed base 11 and located between the two positive lenses 131 so that the two positive lenses 131 are spaced apart.

The above compression unit 13 comprising two positive lenses 131 is merely an example provided by this application and is not a specific limitation on the compression unit 13 of this application. In other embodiments, the compression unit 13 may also include only one positive lens 131. Of course, when the compression unit 13 has only one positive lens 131, there is no need to provide the first spacer ring 14. When the compression unit 13 comprises two positive lenses 131, in order to achieve optical isolation between the two positive lenses 131, in this embodiment, the first spacer ring 14 is specifically provided between the two positive lenses 131. The structural form of the first spacer ring 14 is not specifically limited in this application, as long as it can isolate the two positive lenses 131.

Third, there are a plurality of replacement modules 20. Each replacement module 20 comprises an optical head 21 and a shaping unit disposed inside the optical head 21. The optical head 21 is detachably connectable to the fixed base 11 of the fixed module 10. The shaping unit is used for shaping the light beam emitted from the fixed module 10 and then emitting it.

This application does not limit the optical element form and function of the shaping unit inside the replacement module 20. The optical elements of the shaping unit of each replacement module 20 may be different or the same (when the optical elements are the same, specific parameters such as surface shapes of the same optical elements need to be different), as long as the spot sizes obtained on the receiving surface after passing through the shaping units of different replacement modules 20 are different.

In this embodiment, the light beam emitted from the light source 12 is incident on the replacement module 20 cooperating with the fixed module 10 after fast-axis compression by the fast-axis collimator and compression by the compression unit 13, and is emitted after being shaped by the shaping unit of this replacement module 20. The spot sizes of the light beams emitted through any two different replacement modules 20 on the receiving surface are different.

In summary, the variable spot lens structure provided in this application comprises a fixed module 10 and a plurality of replacement modules 20 configured to cooperate with the fixed module 10. The fixed module 10 comprises a fixed base 11, a light source 12 disposed inside the fixed base 11, and a compression unit 13 fixed on the fixed base 11 and located on a light-emitting side of the light source 12. The plurality of replacement modules 20 are used for being interchangeably disposed on a light-emitting side of the fixed module 10, and each of the replacement modules 20 comprises an optical head 21 and a shaping unit disposed inside the optical head 21. The optical head 21 of each replacement module 20 is detachably connectable to the fixed base 11 of the fixed module 10. A light beam emitted from the light source 12 is incident on the replacement module 20 after compression by the compression unit 13, and is emitted after being shaped by the shaping unit of the replacement module 20. Spot sizes of light beams emitted through any two different replacement modules 20 on a receiving surface are different. In this application, by equipping the fixed module 10 with multiple different replacement modules 20, one set of the variable spot lens structure can achieve output of spots with multiple specification sizes, meeting various needs of users, and having good market application prospects. Compared to existing laser devices that can only output one size of spot, the flexibility of this application is better. Furthermore, the fixed module 10 in this application is provided with the fast-axis collimator and the compression unit 13, so that within the fixed module 10, this application can reduce the divergence angle of the light beam emitted by the light source 12 in the fast-axis direction, making the fast-axis and slow-axis divergence angles similar. While making the fast-axis and slow-axis divergence angles similar, this application also compresses the light beam through the compression unit 13, thereby enabling small-sized spots to be obtained on the receiving surface. Compared to arranging the compression unit 13 inside the replacement module 20, this can further reduce the number of optical elements in the variable spot lens structure, reduce the overall volume of the variable spot lens structure, and achieve cost reduction. Thus, the variable spot lens structure provided in this application can output multiple different specifications of small-sized spots, improving the application flexibility of laser modules.

To facilitate fixing the compression unit 13 to the fixed base 11, optionally, referring to FIG. 4, the fixed module 10 further comprises a first pressing frame 15. The first pressing frame 15 is disposed on a side of the fixed base 11 close to the replacement module 20 and is used for fixing the compression unit 13 to the fixed base 11.

Referring to FIG. 3 and FIG. 4, optionally, the fixed base 11 comprises a mounting seat 111 and a mounting head 112 connected to the mounting seat 111. The light source 12 is disposed inside the mounting seat 111. The compression unit 13 is fixed on the mounting seat 111. The optical head 21 of the replacement module 20 is detachably connected to the mounting head 112.

In this embodiment, a cooling channel is provided inside the mounting seat 111. Thus, the mounting seat 111 can cool down the light source 12 installed inside it, reducing the operating temperature of the entire structure.

Furthermore, the fixed module 10 may further comprise a thermoelectric cooler 16. The mounting head 112 is connected to the mounting seat 111 through the thermoelectric cooler 16. A hot side of the thermoelectric cooler 16 is close to the mounting seat 111, and a cold side thereof is close to the mounting head 112.

When the compression unit 13 comprises two positive lenses 131, the two positive lenses 131 are installed on the mounting seat 111, with the first spacer ring 14 placed in between to ensure the optical spacing, and finally the first pressing frame 15 and screws are used to fix them on the mounting seat 111. The thermoelectric cooler 16 and the mounting head 112 are fixed on the mounting seat 111 by screws. The hot side of the thermoelectric cooler 16 is tightly attached to the mounting seat 111 and dissipates heat through the cooling channel of the mounting seat 111. The cold side of the thermoelectric cooler 16 faces outward, used for transferring the cooling effect to the light exit port, so that when this structure is used for laser medical treatment or aesthetics, it contacts the skin. Thus, when this variable spot lens structure is applied to laser hair removal, it can achieve the purpose of ice point hair removal.

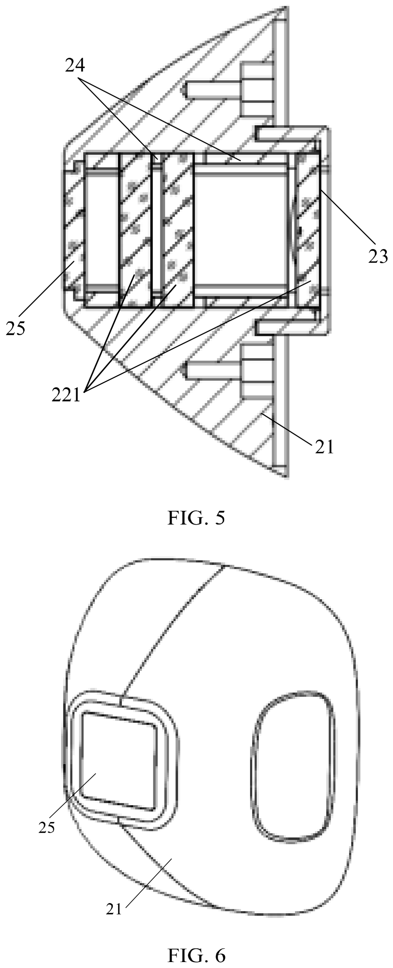

Referring to FIG. 5 to FIG. 11, in this embodiment, the shaping unit comprises at least one optical lens 221. The replacement module 20 further comprises a second pressing frame 23. The second pressing frame 23 is disposed on a side of the replacement module 20 close to the fixed module 10 and is used for fixing the optical lens 221 inside the optical head 21.

The function of the second pressing frame 23 is the same as the principle of the first pressing frame 15, which will not be repeated here. In addition, the optical function of the optical lens 221 is not limited in this application, and those skilled in the art can determine it according to actual needs, for example, it can be a homogenizing element.

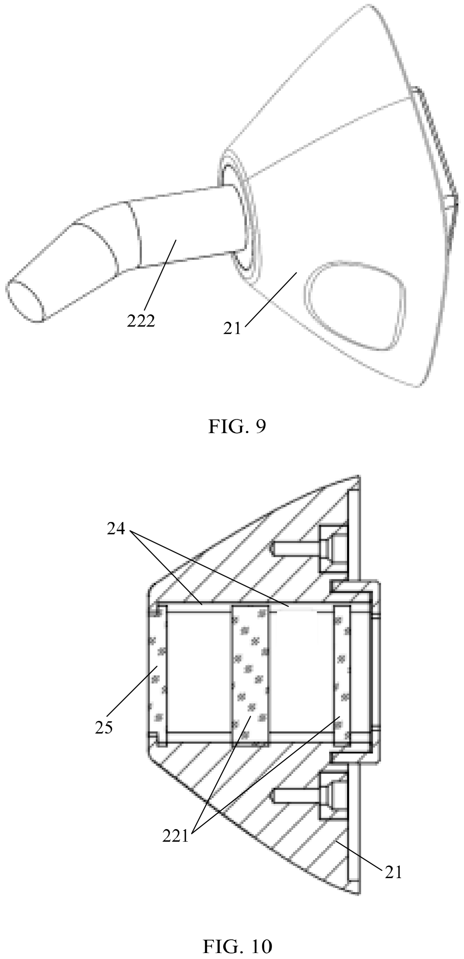

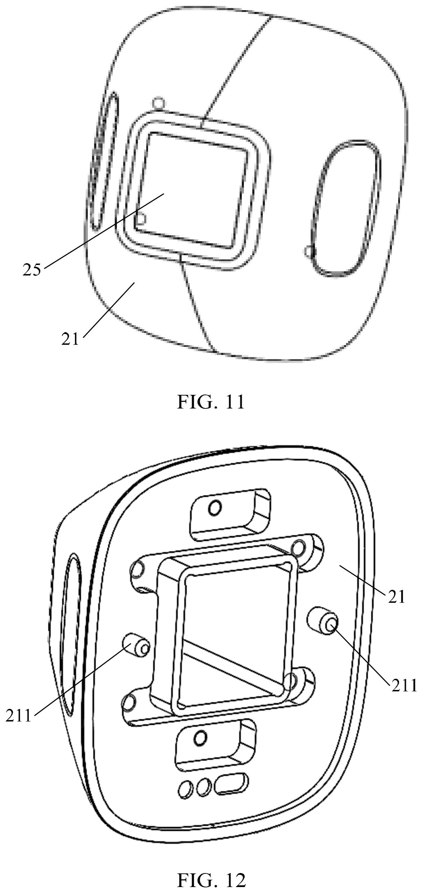

For example, optionally, as shown in FIG. 10 and FIG. 11, the optical lenses 221 comprise at least two. The replacement module 20 further comprises a second spacer ring 24. The second spacer ring 24 is embedded in a second accommodating cavity of the optical head 21 and located between two adjacent optical lenses 221 so that the two adjacent optical lenses 221 are spaced apart.

It should be noted that, in a first feasible implementation, as shown in FIG. 10, the optical lenses 221 may include only two. The replacement module 20 may further include a window plate 25 disposed at the light exit port of the replacement module 20. Thus, a second spacer ring 24 can be placed between the two optical lenses 221, and another second spacer ring 24 can also be placed between the window plate 25 and the optical lens 221 close to it.

For another example, in a second feasible implementation, as shown in FIG. 5 and FIG. 6, the optical lenses 221 may include three. The replacement module 20 may further include a window plate 25 disposed at the light exit port of the replacement module 20. As shown in FIG. 5, when the optical lenses 221 include two, a second spacer ring 24 can be placed between the two optical lenses 221, and another second spacer ring 24 can also be placed between the window plate 25 and the optical lens 221 close to it.

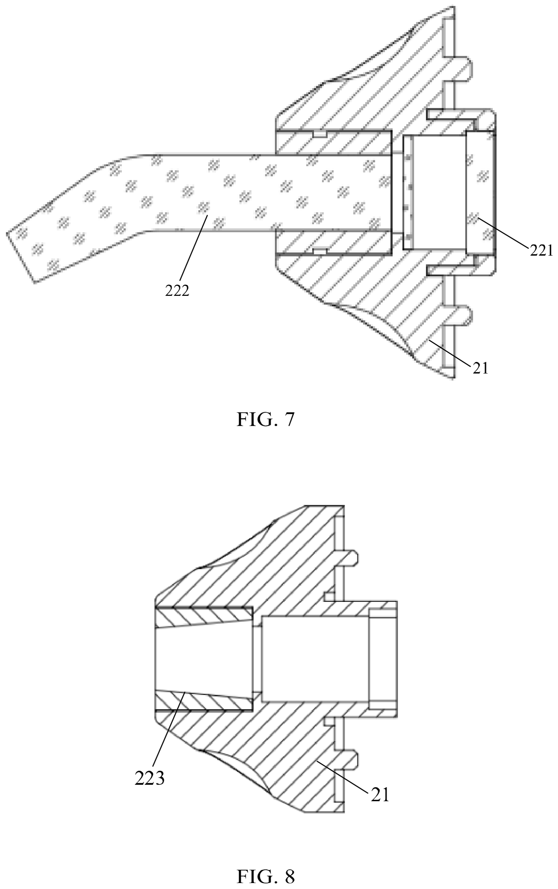

Optionally, the shaping unit includes a light-guiding element. As shown in FIG. 7 and FIG. 9, the light-guiding element can be a light guide rod 222 (i.e., on the basis of including at least one optical lens 221, it may also include a light guide rod 222). The light guide rod 222 penetrates through the optical head 21 and is located on the light-emitting side of the optical lens 221. A light beam emitted from the optical lens 221 can be emitted after passing through the light guide rod 222.

Alternatively, as shown in FIG. 8, the light-guiding element may also be a reflective cavity 223. The reflective cavity 223 penetrates through the optical head 21. A light beam emitted from the fixed module 10 can be emitted after being reflected by the reflective cavity 223.

It should be noted that the above methods are only several examples of the shaping unit of the replacement module 20 provided by this application (i.e., including at least one optical lens 221, or, on the basis of including at least one optical lens 221, also including a light-guiding element), and are not specific limitations of this application. Those skilled in the art can set the shaping unit inside the replacement module 20 according to actual needs.

Also, it should be noted that the fixed base 11 of the fixed module 10 and the optical head 21 of the replacement module 20 are detachably connected. For example, it can be achieved by any one of the following methods:
The fixed base 11 and the optical head 21 are connected by a hinge; or, the fixed base 11 and the optical head 21 are screw-connected; or, the fixed base 11 is provided with a guide groove, the optical head 21 is provided with a guide rail adapted to the guide groove, and the guide rail is slidably insertable into the guide groove; or, the fixed base 11 is provided with a first engagement portion, the optical head 21 is provided with a second engagement portion, and the first engagement portion and the second engagement portion are engaged with each other; or, a surface of the fixed base 11 close to the optical head 21 is provided with a first magnet, a surface of the optical head 21 close to the fixed base 11 is provided with a second magnet, and the first magnet and the second magnet are capable of attracting each other; or, the fixed base 11 is provided with a first thread, the optical head 21 is provided with a second thread, and the first thread and the second thread are threadedly connected.

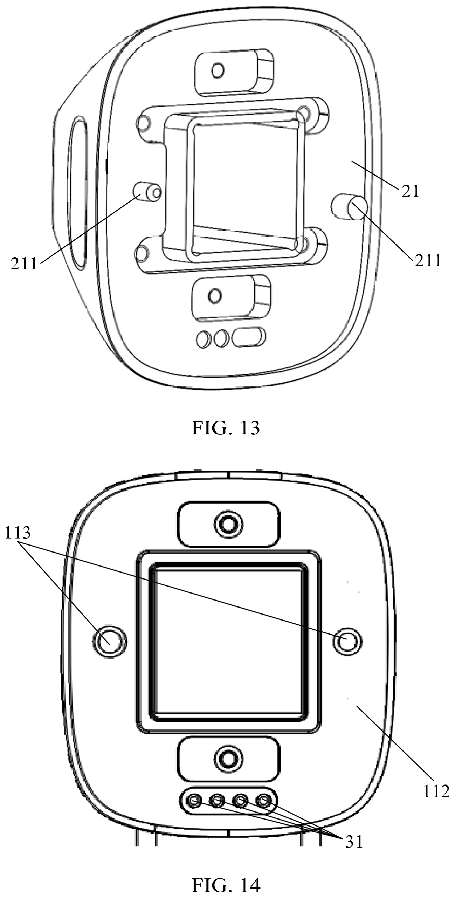

Referring to FIG. 12 and FIG. 13, optionally, a surface of the optical head 21 close to the fixed base 11 is provided with two positioning pins 211. A surface of the fixed base 11 close to the optical head 21 is provided with two positioning holes 113. The two positioning pins 211 and the two positioning holes 113 correspond one-to-one. Diameters of the two positioning pins 211 are different, or, the two positioning pins 211 are arranged asymmetrically about a center of the optical head 21. By providing two positioning pins 211 on the optical head 21 and correspondingly providing two positioning holes 113 on the fixed base 11, and setting the diameters of the two positioning pins 211 to be different, or arranging the two positioning pins 211 asymmetrically about the center of the optical head 21, this application can achieve a fool-proof effect when detachably connecting the optical head 21 and the fixed base 11, preventing reverse installation.

Of course, the positions of the positioning pins 211 and the positioning holes 113 can also be swapped, i.e., two positioning holes 113 are provided on the optical head 21, and two corresponding positioning pins 211 are provided on the fixed base 11.

Furthermore, in this embodiment, in order to facilitate controlling the output light energy of the light source 12 according to the type of the replacement module 20, thereby making the energy of the output light spot controllable, in this embodiment, optionally, the variable spot lens structure further comprises an identification module and a controller. The identification module is used for identifying the type of the replacement module 20 and transmitting it to the controller. The controller is used for controlling the output light energy of the light source 12 according to the type of the replacement module 20.

It should be noted that the controller controlling the output light energy of the light source 12 according to the type of the replacement module 20 can be achieved by controlling the switching to light sources 12 that output different energies. That is, the fixed module 10 can be provided with a variety of light sources 12, each outputting light beams with different energies. The controller can control the switching to the light source 12 corresponding to the current replacement module 20 cooperating with the fixed module 10 according to the type of the replacement module 20 (i.e., drive the corresponding light source 12 to be installed inside the fixed base 11).

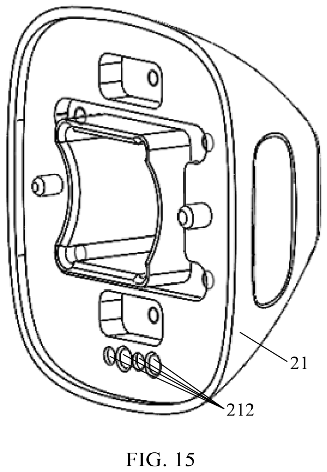

The identification module can be implemented in the following ways: For example, in a first feasible implementation, referring to FIG. 14 and FIG. 15, the identification module comprises a plurality of probes 31 disposed on a surface of the fixed base 11 close to the optical head 21. Each optical head 21 of the replacement modules 20 is provided with a plurality of probe holes 212 corresponding one-to-one with the probes 31. When an optical head 21 of a different replacement module 20 is connected to the fixed base 11 of the fixed module 10, two different probes 31 can be electrically connected and send an identification signal to the controller. The identification signal is different when different replacement modules 20 are connected to the fixed module 10. The controller can determine the type of the replacement module 20 connected to the fixed module 10 according to the identification signal.

It should be noted that the fixed base 11 is distributed with a certain number of probes 31, which are elastic and numbered sequentially. When the replacement module 20 and the fixed module 10 are installed, only two probes 31 are inserted into two probe holes 212 each time, causing the two probes 31 to be conductive, while the other probes 31 correspond to empty positions or insulating materials so that they are not conductive. Thus, replacement modules 20 of different specifications can conduct probes 31 with different numbers, allowing the system to identify different signals and thereby output different laser light source 12 module energies.

When different replacement modules 20 are installed on the fixed module 10, the two conductive probes 31 are different (for example, when a first replacement module 20 is installed on the fixed module 10, a first probe and a second probe may be conductive; when a second replacement module 20 is installed on this module, a second probe and a third probe may be conductive). In this way, the controller can determine the type of the current replacement module 20 according to the conductive state and thus control the output light energy of the light source 12.

In a second feasible implementation, the identification module comprises a pressure sensor disposed on a surface of the fixed base 11 close to the optical head 21. Each optical head 21 of the replacement modules 20 is provided with a different engagement portion. When the optical head 21 of the replacement module 20 is connected to the fixed base 11 of the fixed module 10, the engagement portion presses the pressure sensor. The pressure sensor can send a corresponding pressure signal to the controller. The pressure signal is different when different replacement modules 20 are connected to the fixed module 10. The controller can determine the type of the replacement module 20 connected to the fixed module 10 according to the pressure signal.

That is, besides using the principle of circuit conduction to distinguish identification signals, a pressure sensor can also be installed in the fixed module 10, and different structures (i.e., engagement portions) are designed on the replacement end, so that when installing different models of replacement modules 20, different pressure values can be output, thereby outputting different signals.

Exemplarily, the lengths of the engagement portions of different replacement modules 20 along a first direction may be different, where the first direction is the arrangement direction of the replacement module 20 and the fixed module 10.

In a third feasible implementation, the identification module comprises a signal generator and a detector disposed on the fixed base 11. Each optical head 21 of the replacement modules 20 is provided with a reflector, and a distance from the reflector of each replacement module 20 to the fixed base 11 is different. When the optical head 21 of the replacement module 20 is connected to the fixed base 11 of the fixed module 10, the signal generator emits a signal to the reflector, and the signal is reflected by the reflector and then received by the detector. The controller is used for obtaining a detection signal from the detector and determining the type of the replacement module 20 connected to the fixed module 10 according to the detection signal.

That is, non-contact detection signals can also be used. A signal generator and a detector are installed in the fixed module 10. A reflector is installed on the replacement module 20 at a position corresponding to the signal generator, so that detectors at different positions can receive signals, thereby identifying the model of the replacement module 20.

In another aspect, the invention provides a laser device comprising the aforementioned variable spot lens structure. Since the specific structure and beneficial effects of the aforementioned variable spot lens structure have been elaborated above, they will not be repeated here.

The above descriptions are only preferred embodiments of the invention and are not intended to limit the invention. For those skilled in the art, the invention may have various modifications and changes. Any modification, equivalent replacement, improvement, etc., made within the spirit and principle of the invention shall be included within the protection scope of the invention.

Additionally, it should be noted that the various specific technical features described in the above detailed description can be combined in any suitable manner without conflict. To avoid unnecessary repetition, the invention will not separately explain various possible combinations.

## Claims

1. A variable spot lens structure, **characterized in that** comprising a fixed module and a plurality of replacement modules configured to cooperate with the fixed module, the fixed module comprises a fixed base, a light source disposed inside the fixed base, a fast-axis collimator disposed on a light-emitting side of the light source, and a compression unit fixed on the fixed base and located on a light-emitting side of the fast-axis collimator, the plurality of replacement modules are used for being interchangeably disposed on a light-emitting side of the fixed module, and each of the replacement modules comprises an optical head and a shaping unit disposed inside the optical head,
the optical head of each replacement module is detachably connectable to the fixed base of the fixed module; a light beam emitted from the light source is incident on the replacement module after fast-axis compression by the fast-axis collimator and compression by the compression unit, and is emitted after being shaped by the shaping unit of the replacement module; wherein, spot sizes of light beams emitted through any two different replacement modules on a receiving surface are different.

2. The variable spot lens structure according to claim 1, **characterized in that** the compression unit comprises lenses or lens groups arranged sequentially along an optical axis direction.

3. The variable spot lens structure according to claim 1 or 2, **characterized in that** the fixed module further comprises a first pressing frame, the first pressing frame is disposed on a side of the fixed base close to the replacement module, and is used for fixing the compression unit to the fixed base.

4. The variable spot lens structure according to claim 1, **characterized in that** the fixed base comprises a mounting seat and a mounting head connected to the mounting seat, the light source is disposed inside the mounting seat, the compression unit is fixed on the mounting seat, and the optical head of the replacement module is detachably connected to the mounting head.

5. The variable spot lens structure according to claim 4, **characterized in that** the fixed module further comprises a thermoelectric cooler, the mounting head is connected to the mounting seat through the thermoelectric cooler, a hot side of the thermoelectric cooler is close to the mounting seat, and a cold side thereof is close to the mounting head.

6. The variable spot lens structure according to claim 4, **characterized in that** a cooling channel is provided inside the mounting seat.

7. The variable spot lens structure according to claim 1, **characterized in that** the shaping unit comprises at least one optical lens, and the replacement module further comprises a second pressing frame, the second pressing frame is disposed on a side of the replacement module close to the fixed module, and is used for fixing the optical lens inside the optical head.

8. The variable spot lens structure according to claim 7, **characterized in that** the shaping unit comprises a light-guiding element.

9. The variable spot lens structure according to claim 8, **characterized in that** the light-guiding element is a light guide rod, the light guide rod penetrates through the optical head and is located on a light-emitting side of the optical lens; a light beam emitted from the optical lens can be emitted after passing through the light guide rod.

10. The variable spot lens structure according to claim 8, **characterized in that** the light-guiding element is a reflective cavity, the reflective cavity penetrates through the optical head, a light beam emitted from the fixed module can be emitted after being reflected by the reflective cavity.

11. The variable spot lens structure according to claim 1 or 4, **characterized in that** the fixed base and the optical head are connected by a hinge,
or, the fixed base and the optical head are screw-connected, or, the fixed base is provided with a guide groove, the optical head is provided with a guide rail adapted to the guide groove, and the guide rail is slidably insertable into the guide groove,
or, the fixed base is provided with a first engagement portion, the optical head is provided with a second engagement portion, and the first engagement portion and the second engagement portion are engaged with each other,
or, a surface of the fixed base close to the optical head is provided with a first magnet, a surface of the optical head close to the fixed base is provided with a second magnet, and the first magnet and the second magnet are capable of attracting each other,
or, the fixed base is provided with a first thread, the optical head is provided with a second thread, and the first thread and the second thread are threadedly connected.

12. The variable spot lens structure according to claim 1,
**characterized in that** a surface of the optical head close to the fixed base is provided with two positioning pins, a surface of the fixed base close to the optical head is provided with two positioning holes, and the two positioning pins and the two positioning holes correspond one-to-one; wherein, diameters of the two positioning pins are different, or, the two positioning pins are arranged asymmetrically about a center of the optical head.

13. The variable spot lens structure according to claim 1,
**characterized by** further comprising an identification module and a controller; the identification module is used for identifying a type of the replacement module and transmitting it to the controller, and the controller is used for controlling an output light energy of the light source according to the type of the replacement module.

14. The variable spot lens structure according to claim 13,
**characterized in that** the identification module comprises a plurality of probes disposed on a surface of the fixed base close to the optical head, and each optical head of the replacement modules is provided with a plurality of probe holes corresponding one-to-one with the probes; when an optical head of a different replacement module is connected to the fixed base of the fixed module, two different probes can be electrically connected and send an identification signal to the controller, the identification signal is different when different replacement modules are connected to the fixed module, and the controller can determine the type of the replacement module connected to the fixed module according to the identification signal.

15. The variable spot lens structure according to claim 13,
**characterized in that** the identification module comprises a pressure sensor disposed on a surface of the fixed base close to the optical head, and each optical head of the replacement modules is provided with a different engagement portion; when the optical head of the replacement module is connected to the fixed base of the fixed module, the engagement portion presses the pressure sensor, the pressure sensor can send a corresponding pressure signal to the controller, the pressure signal is different when different replacement modules are connected to the fixed module, and the controller can determine the type of the replacement module connected to the fixed module according to the pressure signal.

16. The variable spot lens structure according to claim 13,
**characterized in that** the identification module comprises a signal generator and a detector disposed on the fixed base, each optical head of the replacement modules is provided with a reflector, and a distance from the reflector of each replacement module to the fixed base is different; when the optical head of the replacement module is connected to the fixed base of the fixed module, the signal generator emits a signal to the reflector and the signal is reflected by the reflector and then received by the detector, and the controller is used for obtaining a detection signal from the detector and determining the type of the replacement module connected to the fixed module according to the detection signal.

17. A laser device, **characterized in that** comprising the variable spot lens structure according to any one of claims 1 to 16.
